# EUROPÄISCHE PATENTSCHRIFT

(11) **EP 3 052 682 B1**
(45) Veröffentlichungstag und Bekanntmachung des Hinweises auf die Patenterteilung: **03.07.2019**
(21) Anmeldenummer: 14722111.3
(22) Anmeldetag: 07.05.2014
(51) Int. Cl.: D01G 27/00, G01N 27/04

(54) **WICKELMASCHINE ZUR ERZEUGUNG VON WATTEWICKELN UND VERFAHREN ZUM WICKELN VON FASERBÄNDERN**
WINDING MACHINE FOR PRODUCING LAP ROLLS AND METHOD FOR WINDING SLIVERS
ENROULEUR POUR LA PRODUCTION DE ROULEAUX DE NAPPE ET PROCÉDÉ D'ENROULAGE DE RUBANS DE FIBRES

(30) Priorität: 01.10.2013 DE 102013110916
(43) Veröffentlichungstag der Anmeldung: 10.08.2016
(73) Patentinhaber: Trützschler GmbH & Co. KG, 41199 Mönchengladbach (DE)
(72) Erfinder: SAEGER, Nicole, 50259 Pulheim (DE); FRIEDRICH, Roland, 41366 Schwalmtal (DE); FELDER, Martin, 41061 Mönchengladbach (DE)
(86) Internationale Anmeldenummer: PCT/EP2014/001209
(87) Internationale Veröffentlichungsnummer: WO 2015/049017

(56) Entgegenhaltungen:
- EP-A2- 0 942 080
- WO-A2-03/050530
- CH-A- 411 400
- DE-A1- 2 845 995
- DE-A1- 4 334 585
- US-A- 2 263 017

## Beschreibung

Die Erfindung betrifft eine Wickelmaschine zum Wickeln von Faserbändern, mit einer Wickeleinheit, auf oder in der der Wickel gebildet wird, wobei die Wickelmaschine mindestens ein Mittel zum Erfassen des Feuchtegehaltes im Fasermaterial aufweist. Weiterhin betrifft die Erfindung ein Verfahren zum Wickeln von Faserbändern.

Die heute übliche Speisung von Flachkämmmaschinen erfolgt durch Wattewickel, die zuvor in Wickelmaschinen aus einzelnen Bändern erzeugt wurden. Dazu erhält die Wickelmaschine die Vorlage in Bandform von mindestens einer Strecke, wobei die Vorlage in runden oder rechteckigen Kannen zwischengespeichert wird. Die Wickelmaschine besteht üblicherweise aus einer Wickeleinheit mit mindestens zwei Wickelwalzen, auf denen mittels einer Hülse der Wattewickel gebildet wird. Der Wickeleinheit vorgelagert sind in der Regel mindestens ein Paar Druckwalzen, die die Bänder umlenken, komprimieren und/oder ggf. verstrecken. Vor den Druckwalzen ist ein Einlaufbereich angeordnet, indem eine weitere Verdichtungseinheit oder ein Glätter angeordnet sein kann.

Die Qualität des erzeugten Wickels ist mit entscheidend für die Produktivität der nachfolgenden Kämmmaschine, sowie die erzeugbare Kammzugqualität und den erforderlichen Kämmlingsanteil. Eine hohe Gleichmäßigkeit, geringe Haarigkeit und ein gutes Abrollverhalten zeichnen einen guten Wickel aus, damit die Kämmmaschine mit möglichst geringem Stillstand läuft. Diese Merkmale können nicht erreicht werden, wenn beim Wickelvorgang durch mitgeführte Luft bei zu hohen Wickelgeschwindigkeiten große Blasen auftreten. Der Wickel wird ungleichmäßig und teilweise sogar unbrauchbar. Mit zunehmender Anzahl an Wattelagen steigt die Blasenbildung während des Wickelprozesses. Die Blasenbildung lässt sich je nach Blasenbild durch einen höheren Druck der Wickelwalzen reduzieren, wodurch sich gleichzeitig das Abrollverhalten des Wickels reduziert. Alternativ lässt sich die Blasenbildung auch durch eine geringere Wickelgeschwindigkeit vermeiden, was sich negativ auf die Produktivität auswirkt. Es ist bekannt, dass das Klima in der Spinnerei einen erheblichen Einfluss auf die Qualität des Prozesses hat und es sind verschiedene Versuche bekannt, das optimale Klima für den einzelnen Verarbeitungsprozess konstant zu halten.

In der EP 1012360 B1 werden Maschinen zur Spinnereivorbereitung oder Teile der Maschinen mit einem Gehäuse ausgestattet, das mit einem geringen Überdruck versehen ist, um Schmutz und Staub dem Verarbeitungsprozess fernzuhalten. Dabei kann der Überdruck mit einer Klimaanlage oder einem normalen Druckgebläse erzeugt werden und Mittel zur Überwachung der Feuchtigkeit und/oder Temperatur aufweisen, wobei der technologische Einfluss eines geregelten Klimas nicht offenbart ist. Nachteilig ist, dass ein erheblicher energetischer Aufwand betrieben wird, um die Fasern in einem konstanten Klima zu verarbeiten.

Die EP 412446 B1 beschreibt in der Einleitung den Einfluss von Temperatur und Feuchtigkeit bei der Verarbeitung von Fasern, wobei beim Messen von Fasern eine gewisse Luftfeuchtigkeit erwünscht ist, diese beim Kardieren aber eher unerwünscht ist. Hierzu sieht die Schrift als Lösung vor, klimatisierte Luft in den Verarbeitungsraum einer Spinnereimaschine zu leiten und die Fasern damit durchströmen zu lassen. Mittels einer Messvorrichtung wird dann die tatsächliche Temperatur und Feuchtigkeit der Fasern gemessen und mit diesen Werten die Klimaanlage geregelt. Auch dieses Verfahren wird mit einem hohen energetischen Aufwand betrieben, den die Betreiber der Spinnereien aufgrund der hohen Energiekosten nicht mehr bereit sind zu zahlen.

In der WO 03/050530 A2 wird ein Verfahren zur Ermittlung der Bandmasse eines bewegten Faserbandes beschrieben, bei dem die Verwendung von Microwellen offenbart wird.

Die CH 411400 offenbart eine Schlagmaschine, bei der ein Faserband durch drei vertikal angeordnete Verdichterwalzen geführt wird, wobei ein Fühler die Banddicke feststellt und in Abhängigkeit der Faserfeuchtigkeit regelt.

In der DE 2845995 A1 wird eine Papiermaschine beschrieben, bei der die Feuchtigkeit über die Breite der Warenbahn eingestellt wird. Hierzu werden vor einem Trockner Walzenpaare angeordnet, die die Feuchtigkeit aus der Warenbahn pressen sollen.

In der DE 4334585 A1 wird eine Wickelmaschine zum Wickeln von Faserbändern für die Kämmereivorbereitung in der Textilindustrie mit einer Wickeleinheit und einer dieser Wickeleinheit unmittelbar vorgeschaltete Behandlungszone, durch welche das zugeführte Fasermaterial hindurchgeführt ist und welche mit Mitteln zum Zuführen von Feuchtigkeit in die Zone versehen ist.

Es ist Aufgabe der Erfindung eine Wickelmaschine zu schaffen, mit der unabhängig von den Klimaverhältnissen ein qualitativ hochwertiger Wickel erzeugt wird. Weiterhin ist es Aufgabe der Erfindung, ein zugehöriges Verfahren zum Wickeln von Faserbändern zu schaffen.

Die Erfindung löst die gestellte Aufgabe durch die Lehre nach Anspruch 1 und 9; weitere vorteilhafte Ausgestaltungsmerkmale der Erfindung sind durch die Unteransprüche gekennzeichnet.

Gemäß der technischen Lehre nach Anspruch 1 umfasst die Wickelmaschine zum Wickeln von Faserbändern mindestens eine Wickeleinheit, auf oder in der der Wickel gebildet wird, wobei die Wickelmaschine mindestens ein Mittel zum Erfassen des Feuchtegehaltes im Fasermaterial aufweist.

Die Erfindung ist dadurch gekennzeichnet, dass das mindestens eine Mittel den Feuchtegehalt des Fasermaterials als Regelgröße ermittelt, ein Regler oder eine Steuerung die Regelgröße mit einer Führungsgröße vergleicht und bei Abweichung von der Führungsgröße ein Signal erzeugt, durch das mindestens ein Stellglied zur Änderung der Betriebsparameter der Wickelmaschine betätigbar ist. Hierzu ist vorgesehen, dass das Stellglied als ein Antriebsmotor und/oder als ein Zylinder zur Einstellung des Wickeldruckes und/oder als ein einstellbares Element eines Vorverdichters ausgebildet ist.

Im Gegensatz zum Stand der Technik wird damit kein Mikroklima angepasst, sondern die Betriebsparameter der Wickelmaschine passen sich den klimatischen Umgebungsbedingungen an, so dass immer ein qualitativ hochwertiger Wickel erzeugt wird. Dabei kann bewusst in Kauf genommen werden, dass die Produktivität der Wickelmaschine bei einem zu trockenen Raumklima sinkt. Dies kann durch einen erhöhten Druck der Wickelwalzen oder ein stärkeres Einwirken des Vorverdichters kompensiert werden. Im Hinblick auf die gesamten Betriebskosten der Wickelmaschine ist dies aber deutlich günstiger, als der energetische Aufwand, der für die Erzeugung eines stör- und wartungsanfälligen Mikroklimas aufgewendet werden muss. Mit der Anpassung der Betriebsparameter an das vorhandene Raum- oder Umgebungsklima kann der Wickler so gefahren werden, dass die für eine schlechte Wickelqualität verantwortliche Blasenbildung erst sehr spät oder gar nicht einsetzt.

Weitere vorteilhafte Ausführungsformen sind durch die Unteransprüche gekennzeichnet.

In einer vorteilhaften Ausführungsform ist das Mittel zur Bestimmung des Feuchtegehaltes als optisches oder strahlenbasiertes Meßsystem ausgebildet. Damit ist eine berührungslose Messung innerhalb der gesamten Wickelmaschine unabhängig von der Wickelgeschwindigkeit möglich. Die Anordnung kann dabei an den Tischkalanderwalzen, im Einlaufbereich, an den Druckwalzen oder an der Oberfläche der Wickelwalzen erfolgen. Alternativ kann das Mittel als elektrische Widerstandsmeßeinrichtung ausgebildet sein, die im oder vor dem Einlaufbereich angeordnet ist.

Besonders bevorzugt ist die elektrische Widerstandsmeßeinrichtung in die Tischkalanderwalzen oder in die Druckwalzen integrierbar, wobei der elektrische Widerstand des Fasermaterials bestimmbar ist. Es ergibt sich eine ausreichend lange Regelstrecke, um den nachfolgenden Vorverdichter oder die Wickelwalzen einzustellen.

Ebenfalls ist eine derartige Anordnung des Meßsystems möglich, bei der während des Aufwickelvorganges die Feuchtigkeit des Fasermaterials an der Wickeloberfläche oder an der Oberfläche der Wickelwalze bestimmt wird.

In einer weiteren Ausführungsform kann das Stellglied als Antriebsmotor der Wickelwalzen ausgebildet sein, mit denen die Wickelgeschwindigkeit veränderbar ist. Bei einem zu trockenen Klima mit geringer Luftfeuchtigkeit kann dadurch die Wickelgeschwindigkeit reduziert werden, wodurch die Blasenbildung abnimmt.

Alternativ kann das Stellglied als Zylinder zur Einstellung des Wickeldruckes ausgebildet sein. Sinkt die Feuchtigkeit des Fasermaterials, kann über eine Druckerhöhung bzw. eine Vergrößerung des Anpressdruckes auf den Wickel die Blasenbildung reduziert werden.

In einer weiteren vorteilhaften Alternative kann das Stellglied als ein Element eines Vorverdichters ausgebildet sein, mit dem der Widerstand auf das Faserband innerhalb des Vorverdichters veränderbar ist. So können die Elemente des Vorverdichters als Stege ausgeführt sein, an denen das Faserband umgelenkt und geglättet bzw. verdichtet wird. Eine Verstellbarkeit der Stege so zueinander, dass diese mehr verzahnen und damit eine größere Umlenkung des Faserbandes innerhalb des Vorverdichters erfolgt, kann ebenfalls die Qualität des Wickels positiv beeinflussen.

Ebenfalls als Stellglied ausgebildet können die Antriebe der Wickelwalzen, die Antriebe der Druckwalzen, die Antriebe der Tischkalander und die Antriebe des vorgelagerten Streckwerkes sein. Damit lässt sich der Verzug zwischen dem Gatter und dem Streckwerk, der Verzug zwischen dem Streckwerk und dem Tischkalander, der Verzug zwischen dem Tischkalander und den Druckwalzen, sowie der Verzug zwischen den Druckwalzen und den Wickelwalzen einstellen. Weitere Möglichkeiten zur Beeinflussung der Wickelqualität kann die Änderung des Umschlingungswinkels und/oder des Druckes der Druckwalzen oder des Umschlingungswinkels an den Wickelwalzen sein.

Selbstverständlich kann auch eine Kombination der zuvor beschriebenen Maßnahmen die Qualität des Wickels beeinflussen.

Weiterhin weist die Wickelmaschine eine Datenbank auf, in der für verschiedene Fasermaterialien die optimalen Klimadaten und deren Schwellwerte für das Auslösen eines Signals für ein Stellglied zur Änderung der Betriebsparameter hinterlegt sind.

Das erfindungsgemäße Verfahren zum Wickeln von Faserbändern zeichnet sich dadurch aus, dass mindestens der Feuchtegehalt im Fasermaterial bestimmt und damit eine Regelgröße erzeugt wird, wobei die Regelgröße mit einer Führungsgröße verglichen wird und bei Abweichung von der Führungsgröße mittels eines Signals ein Stellglied zur Änderung der Betriebsparameter betätigbar ist. Das Verfahren kann manuell, teilautomatisch oder automatisch erfolgen.

Dabei ist das Stellglied als ein Antriebsmotor und/oder als ein Zylinder zur Einstellung des Wickeldruckes und/oder als ein einstellbares Element eines Vorverdichters ausgebildet. Weitere veränderbare Parameter, die einen Einfluss auf die Qualität des Wickels haben, sind die Antriebe der Wickelwalzen, die Antriebe der Druckwalzen, die Antriebe der Tischkalander und die Antriebe des vorgelagerten Streckwerkes. Damit lässt sich der Verzug zwischen dem Gatter und dem Streckwerk, der Verzug zwischen dem Streckwerk und dem Tischkalander, der Verzug zwischen dem Tischkalander und den Druckwalzen, sowie der Verzug zwischen den Druckwalzen und den Wickelwalzen einstellen. Weitere Möglichkeiten zur Beeinflussung der Wickelqualität kann die Änderung des Umschlingungswinkels und/oder des Druckes der Druckwalzen oder des Umschlingungswinkels an den Wickelwalzen sein. Damit können die wesentlichen Betriebsparameter, die einen Einfluss auf die Qualität des Wickels haben, einzeln oder in Kombination verändert werden.

Damit nicht permanent die Betriebsparameter der Wickelmaschine angepasst werden müssen, wird das Signal zur Betätigung eines Stellgliedes erst dann erzeugt, wenn die Regelgröße einen Schwellwert über- oder unterschritten hat. Damit können kleine Änderungen des Klimas, beispielsweise wenn über eine geöffnete Hallentür kurzfristig Material zu- oder abtransportiert wird, vernachlässigt werden.

In vorteilhafter Ausführungsform ist zusätzlich zum Feuchtegehalt im Fasermaterial auch die Temperatur des Fasermaterials bestimmbar.

Das Signal zur Änderung der Betriebsparameter kann bei einem Unter- oder Überschreiten der Führungsgröße die Wickelgeschwindigkeit verändern. So kann bei zu trockenem Klima und zu geringer Feuchtigkeit des Faserbandes die Wickelgeschwindigkeit von beispielsweise 180 m/min auf 150 m/min gesenkt werden, wodurch die Blasenbildung verringert bzw. vermieden und eine gleichbleibende Wickelqualität gehalten wird.

Alternativ kann bei einem Unter- oder Überschreiten der Führungsgröße der Wickeldruck verändert werden, indem beispielsweise bei zu trockenem Klima der Wickeldruck erhöht wird, was auch wieder die Blasenbildung reduziert bzw. vermeidet.

Zusätzlich oder alternativ kann bei einem Unter- oder Überschreiten der Führungsgröße der Widerstand innerhalb des Vorverdichters verändert werden, indem beispielsweise durch eine stärkere Verzahnung der Stege der Widerstand auf das Faserband erhöht wird, wodurch dieses stärker geglättet wird.

Die Erfindung wird nachfolgend anhand eines Ausführungsbeispieles näher erläutert. Es zeigt:

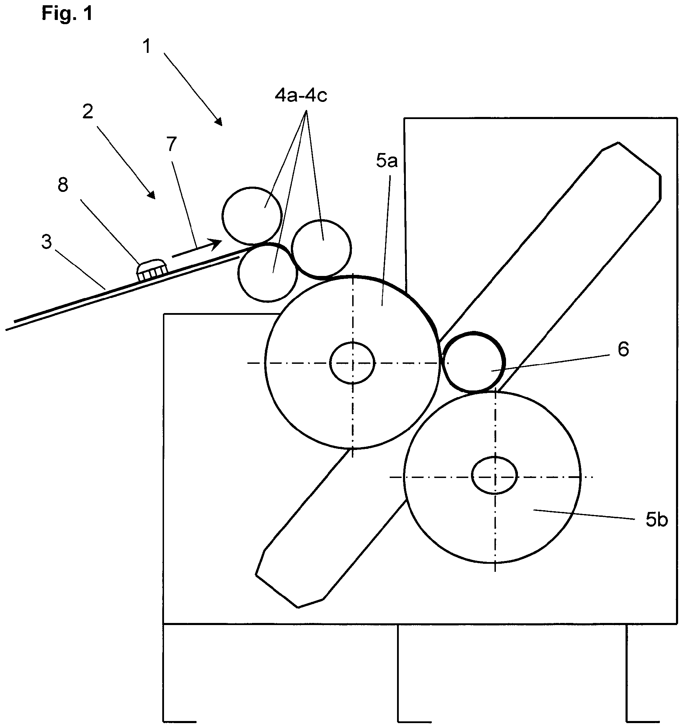

Figur 1: eine schematische Schnittdarstellung einer Wickelmaschine.

In Figur 1 ist eine Wickelmaschine 1 dargestellt, wie sie beispielsweise in der Kämmereivorbereitung in der Textilindustrie verwendet wird. Mehrere Faserbänder 3, die aus natürlichen oder synthetischen Fasern bestehen können, werden über Kannen der Wickelmaschine 1 zugeführt und in einem nicht dargestellten Streckwerk vergleichmäßigt. Die Faserbänder 3 werden weiter über einen Einlaufbereich 2 zu mehreren Druckwalzen 4a - 4c geleitet, die die Faserbänder 3 in den Einzugsbereich zwischen zwei Wickelwalzen 5a, 5b und einer Wickelhülse 6 zur Herstellung eines Wattewickels leiten. Im oder am Einlaufbereich 2 ist ein Vorverdichter 8 angeordnet, der das Faserband 3 vergleichmäßigt. Nicht dargestellt sind die im Einlaufbereich angeordneten Tischkalander, das vor dem Tischkalander angeordnete Streckwerk sowie das vor der Wickelmaschine angeordnete Gatter. Der Wickeldruck wird duch seitlich angeordnete Zylinder, die in Figur 1 nicht dargestellt sind, erzeugt, indem die Wickelhülse 6 gegen die Wickelwalzen 5a, 5b gezogen bzw. gedrückt werden.

Alternativ zu dieser Ausführungsform mit den zwei Wickelwalzen 5a, 5b sind Wickelmaschinen bekannt, bei denen der Wickel innerhalb eines umlaufenden Bandes oder durch ein Band mit einer Wickelwalze gebildet wird. Das Verfahren zur Herstellung des Wattewickels ist für die Erfindung aber nicht relevant.

Erfindungsgemäß weist die Wickelmaschine Mittel zum Erfassen des Feuchtegehaltes im Fasermaterial in der Wickelmaschine auf. In Abhängigkeit von dem ermittelten Feuchtegehalt im Fasermaterial ist der Druck der Wickelwalzen 5a, 5b, die Einstechtiefe des Vorverdichters 8 und/oder die Wickelgeschwindigkeit anpassbar. Weitere einstellbare Parameter können die Antriebe der Wickelwalzen, die Antriebe der Druckwalzen, die Antriebe der Tischkalander und die Antriebe des vorgelagerten Streckwerkes sein. Damit lässt sich der Verzug zwischen dem Gatter und dem Streckwerk, der Verzug zwischen dem Streckwerk und dem Tischkalander, der Verzug zwischen dem Tischkalander und den Druckwalzen, sowie der Verzug zwischen den Druckwalzen und den Wickelwalzen einstellen. Weitere Möglichkeiten zur Beeinflussung der Wickelqualität kann die Änderung des Umschlingungswinkels und/oder des Druckes der Druckwalzen oder des Umschlingungswinkels an den Wickelwalzen sein.

Als optimale Feuchtigkeit für Baumwolle hat sich ein Spinnklima in der Spinnerei von 60 % Luftfeuchtigkeit bei beispielsweise einer Temperatur von 24° C herausgestellt, wobei die Materialfeuchte der entscheidende Faktor ist. Es hat sich herausgestellt, dass eine zu geringe Feuchtigkeit des Fasermaterials einen negativen Einfluss auf die Blasenbildung während des Wickelprozesses hat, wodurch das Lagenbild des Wickels beeinflusst wird, indem beispielsweise verstärkt Verwerfungen auftreten. Ist die Feuchtigkeit zu gering, nimmt die Härte des Wickels ab und das Aufspringen des Wickels kann um etwa 1/3 zu nehmen. Weiterhin sind das Abrollverhalten und die Haarigkeit des Wickels schlechter, als bei einer höheren Luftfeuchtigkeit. Die Erfindung macht sich dabei die Erkenntnis zunutze, dass beispielsweise Baumwolle in einem feuchten Zustand besser zu verarbeiten ist, als in einem trockenen Zustand, da die Festigkeit steigt, gleichzeitig die Fasersteifigkeit und Reibung abnimmt. In Abhängigkeit von der Feuchtigkeit des zu verarbeitenden Materials erfolgt die Einstellung der Wickelmaschine, in dem die Wickelgeschwindigkeit, der Wickeldruck und/oder die Einstechtiefe des Vorverdichters und/oder der Verzug des Faserbandes zwischen den einzelnen Komponenten optimiert werden. Dies kann bei der Verarbeitung von Baumwolle anders erfolgen, als bei der Verarbeitung von Fasermischungen mit beispielsweise einem hohen Anteil an Polyesterfasern.

Bei der Verarbeitung von Polyesterfasern ist eine noch höhere Luftfeuchtigkeit wünschenswert, da mit steigender Luftfeuchtigkeit die statische Aufladung der Fasern abnimmt und diese damit besser verarbeitet werden können.

Bei einer zu geringen Feuchtigkeit des Fasermaterials kann beispielsweise die Wickelgeschwindigkeit von beispielsweise 180 m/min auf 150 m/min heruntergefahren werden, um die Qualität des Wickels zu gewährleisten. Alternativ oder ergänzend kann der Druck der Wickelwalzen 5a, 5b von beispielsweise 6 bar auf 8 bar hochgefahren werden. In weiterer Alternative oder ergänzend können die Stege im Vorverdichter um beispielsweise 5 mm tiefer miteinander verzahnen, so dass das Faserband stärker ausgerichtet und geglättet wird.

In einer vorteilhaften Ausführungsform können als Messgeräte optische Messwertaufnehmer verwendet werden, deren abgestrahltes Licht von der Materialfeuchte absorbiert wird. Alternativ können auch mikrowellenbasierte Meßsysteme verwendet werden, die die Materialfeuchtigkeit messen.

Eine in die Wickelmaschine besonders gut integrierbare Lösung sieht vor, die Materialfeuchte über elektrische Widerstandsmessung zu bestimmen, wobei die Messeinrichtung beispielsweise in den Tischkalanderwalzen oder in den Druckwalzen integriert wird. Damit ergibt sich eine ausreichende Regelstrecke, um beispielsweise die Wickelgeschwindigkeit, den Wickeldruck und/oder die Einstechtiefe des Vorverdichters und/oder den Verzug zu regeln.

Die Anordnung der Messgeräte kann grundsätzlich an oder nach den Tischkalanderwalzen, beispielsweise im Einlaufbereich 2 erfolgen oder an der Wickeloberfläche der Wickelwalzen 5a, 5b.

Generell können die klimatischen Verhältnisse nur individuell vom Kunden ermittelt und beeinflusst werden. Dabei ist die Einhaltung eines optimalen Verarbeitungsklimas für alle Prozessschritte gar nicht oder nur schwer realisierbar. Im Unterschied zum Stand der Technik, bei dem für jeden Verarbeitungsprozess ein energetisch aufwendiges Mikroklima geschaffen wird, geht die Erfindung weiter und passt dabei die Maschinenparameter an das vorhandene Spinnklima an. Die voreingestellten Maschinenparameter können erst dann verändert werden, wenn ein sogenannter Schwellwert unterschritten oder überschritten wird. Beispielsweise bei einer Absenkung der relativen Luftfeuchtigkeit von 60 % auf unter 40 % kann die Wickelgeschwindigkeit abgesenkt werden und/oder gleichzeitig der Wickeldruck erhöht werden. Bei einer Absenkung der relativen Luftfeuchtigkeit von beispielsweise 60 % auf 55 % und dann auf 50 % kann alternativ die Einstechtiefe der Stege des Vorverdichters schrittweise erhöht werden, ohne dass die Produktionskapazität gesenkt wird. Die Regelung bzw. Steuerung über einen Schwellwert hat den Vorteil, dass der Bediener der Wickelmaschine keine Sicherheiten bei der Auswahl der Maschinenparameter berücksichtigen muss, sondern kann dem Material angepasste optimale Parameter auswählen. Damit kann bei optimalem Spinnklima eine höhere Wickelqualität oder eine erhöhte Produktionsgeschwindigkeit erzielt werden

So können in Abhängigkeit der zu verarbeitenden Fasern und der klimatischen Änderungen einerseits die Maschinenparameter zusammen oder einzeln geändert werden, um eine ausreichende Produktqualität zu gewährleisten. Andererseits ist damit gewährleistet, dass bei optimalem Klima die Wickelmaschine mit der höchsten Produktivität fährt.

In bevorzugter Ausführungsform weist die Wickelmaschine eine Datenbank auf, in der für verschiedene Faserarten die optimalen Klimadaten hinterlegt sind. Weiterhin kann in der Datenbank auch der zugehörige Steuerungsalgorithmus hinterlegt sein, mit welchen Einstellungen bei einer Abweichung von den optimalen Klimadaten die Wickelmaschine fährt. Ebenso können die Schwellwerte für jede Faserart hinterlegt werden, die unter- bzw. überschritten werden müssen, damit die Wickelmaschine mit anderen Betriebsparametern fährt.

Die Datenbank kann Teil einer Steuerung/Regelung sein, in der als Eingangsgröße die gemessene Luftfeuchtigkeit als Regelgröße einfließt, mit der optimalen Luftfeuchtigkeit als Führungsgröße verglichen wird und die Steuerung/Regelung mittels eines Signals ein Stellglied betätigt, das beispielsweise auf die Drehzahl der Antriebsmotoren oder auf die Druckzylinder der Wickelwalzen 5a, 5b einwirkt.

Damit ist es möglich, das Klima in einer Spinnerei an die wesentlichen Maschinen, beispielsweise auf die Karden oder Kämmmaschinen, anzupassen, und gleichzeitig Wickel mit optimaler Qualität und Produktivität herzustellen. Auch wenn ggf. aufgrund nicht optimalen Klimas die Produktivität der Wickelmaschine sinkt, sind die gesamten Betriebskosten doch deutlich geringer, als mit dem energetischen Aufwand eines Mikroklimas.

### Bezugszeichen

- 1: Wickelmaschine
- 2: Einlaufbereich
- 3: Faserband
- 4a - 4c: Druckwalzen
- 5a, 5b: Wickelwalze
- 6: Wickelhülse
- 7: Materialflussrichtung
- 8: Vorverdichter

## Patentansprüche

1. Wickelmaschine zum Wickeln von Faserbändern für die Kämmereivorbereitung in der Textilindustrie, mit einer Wickeleinheit, auf oder in der ein Wickel gebildet wird, **dadurch gekennzeichnet, dass** die Wickelmaschine mindestens ein Mittel zum Erfassen des Feuchtegehaltes im Fasermaterial aufweist, wobei das mindestens eine Mittel den Feuchtegehalt des Fasermaterials als Regelgröße ermittelt, ein Regler oder eine Steuerung die Regelgröße mit einer Führungsgröße vergleicht und bei Abweichung von der Führungsgröße ein Signal erzeugt, durch das mindestens ein Stellglied zur Änderung der Betriebsparameter der Wickelmaschine betätigbar ist, wobei das Stellglied als ein Antriebsmotor und/oder als ein Zylinder zur Einstellung des Wickeldruckes und/oder als ein einstellbares Element eines Vorverdichters ausgebildet ist.

2. Wickelmaschine nach Anspruch 1, **dadurch gekennzeichnet, dass** das Mittel als optisches oder strahlenbasiertes Meßsystem ausgebildet ist.

3. Wickelmaschine nach Anspruch 1, **dadurch gekennzeichnet, dass** das Mittel als elektrische Widerstandsmeßeinrichtung ausgebildet ist.

4. Wickelmaschine nach Anspruch 2, **dadurch gekennzeichnet, dass** die Wickelmaschine Tischkalanderwalzen, einen Einlaufbereich, Druckwalzen und Wickelwalzen aufweist, wobei das Meßsystem an den Tischkalanderwalzen, im Einlaufbereich (2), an den Druckwalzen (4a - 4c) oder an der Oberfläche der Wickelwalzen (5a, 5b) angeordnet ist.

5. Wickelmaschine nach Anspruch 3, **dadurch gekennzeichnet, dass** die Wickelmaschine Tischkalanderwalzen und/oder Druckwalzen (4a - 4c) aufweist und dass die Messeinrichtung in die Tischkalanderwalzen oder in die Druckwalzen (4a - 4c) integrierbar ist.

6. Wickelmaschine nach Anspruch 1, **dadurch gekennzeichnet, dass** die Wickelmaschine Wickelwalzen (5a, 5b) aufweist und das Stellglied als Antriebsmotor der Wickelwalzen (5a, 5b) ausgebildet ist, mit denen die Wickelgeschwindigkeit veränderbar ist.

7. Wickelmaschine nach Anspruch 1, **dadurch gekennzeichnet, dass** durch das Element des Vorverdichters (8) der Widerstand auf das Faserband (3) innerhalb des Vorverdichters (8) veränderbar ist.

8. Wickelmaschine nach Anspruch 1, **dadurch gekennzeichnet, dass** die Wickelmaschine Wickelwalzen (5a, 5b) und/oder Druckwalzen (4a - 4c) und/oder Tischkalander aufweist und dass das Stellglied als Antriebsmotor der Wickelwalzen, und/oder der Druckwalzen, und/oder der Tischkalander und/oder eines vorgelagerten Streckwerkes ausgebildet ist, wodurch der Verzug des Faserbandes zwischen diesen Komponenten einstellbar ist.

9. Verfahren zum Wickeln von Faserbändern für die Kämmereivorbereitung in der Textilindustrie, mit einer Wickeleinheit, auf oder in der ein Wickel gebildet wird, **dadurch gekennzeichnet, dass** mindestens der Feuchtegehalt im Fasermaterial bestimmt und damit eine Regelgröße erzeugt wird, wobei die Regelgröße mit einer Führungsgröße verglichen wird und bei Abweichung von der Führungsgröße mittels eines Signals ein Stellglied zur Änderung der Betriebsparameter betätigbar ist, wobei das Stellglied als ein Antriebsmotor und/oder als ein Zylinder zur Einstellung des Wickeldruckes, und/oder als ein einstellbares Element eines Vorverdichters (8) ausgebildet ist.

10. Verfahren nach Anspruch 9, **dadurch gekennzeichnet, dass** das Signal zur Betätigung eines Stellgliedes erst dann erzeugt wird, wenn die Regelgröße einen Schwellwert über- oder unterschritten hat.

11. Verfahren nach Anspruch 9, **dadurch gekennzeichnet, dass** zusätzlich zum Feuchtegehalt im Fasermaterial auch die Temperatur des Fasermaterials bestimmbar ist.

12. Verfahren nach einem der vorhergehenden Ansprüche 9 bis 11, **dadurch gekennzeichnet, dass** bei einem Unter- oder Überschreiten der Führungsgröße die Wickelgeschwindigkeit veränderbar ist.

13. Verfahren nach einem der vorhergehenden Ansprüche 9 bis 12, **dadurch gekennzeichnet, dass** bei einem Unter- oder Überschreiten der Führungsgröße der Wickeldruck veränderbar ist.

14. Verfahren nach einem der vorhergehenden Ansprüche 9 bis 13, **dadurch gekennzeichnet, dass** bei Unter- oder Überschreiten der Führungsgröße der Widerstand innerhalb des Vorverdichters (8) veränderbar ist.

## Claims

1. A winding machine for winding fibre bands for the preparation of combing works in the textile industry, with a winding unit, on or in which a lap is formed, **characterized in that** the winding machine includes at least one means for detecting the humidity content in the fibre material, wherein the at least one means determines the humidity content of the fibre material as a controlled variable, a controller or a control compares the controlled variable to a reference variable, and in case of a deviation from the reference variable produces a signal by means of which an actuator for changing the operational parameters of the winding machine is actuatable, wherein the actuator is formed as a drive motor and/or as a cylinder for adjusting the winding pressure and/or as an adjustable element of a pre-condenser.

2. The winding machine according to claim 1, **characterized in that** the means is formed as an optical measuring system or as a measuring system based on radiation.

3. The winding machine according to claim 1, **characterized in that** the means is formed as an electrical resistance measuring device.

4. The winding machine according to claim 2, **characterized in that** the winding machine includes table calender rolls, an intake area, pressure rolls and winding rolls, wherein the measuring system is disposed at the table calender rolls, in the intake area (2), at the pressure rolls (4a to 4c) or at the surface of the winding rolls (5a, 5b).

5. The winding machine according to claim 3, **characterized in that** the winding machine includes table calender rolls and/or pressure rolls (4a to 4c) and **in that** the measuring device may be incorporated into the table calender rolls or into the pressure rolls (4a - 4c).

6. The winding machine according to claim 1, **characterized in that** the winding machine includes winding rolls (5a, 5b), and the actuator is formed as the drive motor of the winding rolls (5a, 5b), by means of which the winding speed is variable.

7. The winding machine according to claim 1, **characterized in that** the resistance onto the fibre band (3) within the pre-condenser (8) is variable by means of the element of the pre-condenser (8).

8. The winding machine according to claim 1, **characterized in that** the winding machine includes winding rolls (5a, 5b) and/or pressure rolls (4a to 4c) and/or table calenders, and **in that** the actuator is formed as a drive motor of the winding rolls and/or of the pressure rolls and or of the table calenders and/or of an upstream drafting system, whereby the draft of the fibre band is adjustable between said components.

9. A method for winding fibre bands for the preparation of combing works in the textile industry, with a winding unit, on or in which a lap is formed, **characterized in that** at least the humidity content in the fibre material is determined, and thereby a controlled variable is produced, wherein the controlled variable is compared to a reference variable, and in case of a deviation from the reference variable an actuator for changing the operational parameter is actuatable by means of a signal, wherein the actuator is formed as a drive motor and/or as a cylinder for adjusting the winding pressure and/or as an adjustable element of a pre-condenser (8).

10. The method according to claim 9, **characterized in that** the signal for actuating an actuator is only produced if the controlled variable exceeds or falls below a threshold value.

11. The method according to claim 9, **characterized in that** in addition to the humidity content in the fibre material also the temperature of the fibre material may be determined.

12. The method according to any of the preceding claims 9 to 11, **characterized in that** upon exceeding or falling below the reference variable the winding speed is variable.

13. The method according to any of the preceding claims 9 to 12, **characterized in that** upon exceeding or falling below the reference variable the winding pressure is variable.

14. The method according to any of the preceding claims 9 to 13, **characterized in that** upon exceeding or falling below the reference variable the resistance within the pre-condenser (8) is variable.

## Revendications

1. Enrouleur pour enrouler des bandes de fibres pour la préparation au peignage dans l'industrie du textile, avec une unité d'enroulement sur ou dans laquelle un rouleau de nappe est formé, **caractérisé en ce que** l'enrouleur comprend au moins un moyen pour détecter la teneur en humidité dans la matière de fibres, ledit au moins un moyen déterminant la teneur en humidité de la matière de fibres comme valeur de réglage, un régulateur ou une commande comparant la valeur de réglage à une valeur de référence et produisant un signal lors d'une déviation de la valeur de référence, avec lequel au moins un actionneur étant actionnable pour modifier les paramètres opérationnels de l'enrouleur, l'actionneur étant aménagé comme moteur d'entraînement et/ou comme cylindre pour l'ajustage de la pression d'enroulement et/ou comme élément ajustable d'un pré-condenseur.

2. Enrouleur selon la revendication 1, **caractérisé en ce que** le moyen est aménagé comme système de mesure optique ou basé sur rayonnement.

3. Enrouleur selon la revendication 1, **caractérisé en ce que** le moyen est aménagé comme dispositif de mesure de résistance électrique.

4. Enrouleur selon la revendication 2, **caractérisé en ce que** l'enrouleur comprend des rouleaux à table de calandrage, une région d'entrée, des rouleaux de pression et des rouleaux d'enroulement, le système de mesure étant agencé sur les rouleaux à table de calandrage, dans la région d'entrée (2), sur les rouleaux de pression (4a à 4c) ou à la surface des rouleaux d'enroulement (5a, 5b).

5. Enrouleur selon la revendication 3, **caractérisé en ce que** l'enrouleur comprend des rouleaux à table de calandrage et/ou des rouleaux de pression (4a à 4c) et **en ce que** le dispositif de mesure peut s'intégrer dans les rouleaux à table de calandrage ou dans les rouleaux de pression (4a à 4c).

6. Enrouleur selon la revendication 1, **caractérisé en ce que** l'enrouleur comprend des rouleaux d'enroulement (5a, 5b) et l'actionneur est aménagé comme moteur d'entraînement des rouleaux d'enroulement (5a, 5b) par l'intermédiaire desquels la vitesse d'enroulement est variable.

7. Enrouleur selon la revendication 1, **caractérisé en ce que** la résistance sur la bande de fibres (3) à l'intérieur du pré-condenseur (8) est variable par l'élément du pré-condenseur (8).

8. Enrouleur selon la revendication 1, **caractérisé en ce que** l'enrouleur comprend des rouleaux d'enroulement (5a, 5b) et/ou des rouleaux de pression (4a à 4c) et/ou la table de calandrage, et **en ce que** l'actionneur est aménagé comme moteur d'entraînement des rouleaux d'enroulement et/ou des rouleaux de pression et/ou de la table de calandrage et/ou d'un système d'étirage logé en amont, qui font que l'étirage de la bande de fibres est ajustable entre ces composants.

9. Méthode pour enrouler des bandes de fibres pour la préparation au peignage dans l'industrie du textile, avec une unité d'enroulement sur ou dans laquelle un rouleau de nappe est formé, **caractérisée en ce qu'**au moins la teneur en humidité dans la matière de fibres est déterminée et une valeur de réglage est ainsi générée, la valeur de réglage étant comparée à une valeur de référence et, lors d'une déviation de la valeur de référence un actionneur étant actionnable au moyen d'un signal pour modifier les paramètres opérationnels, l'actionneur étant aménagé comme moteur d'entraînement et/ou comme cylindre pour l'ajustage de la pression d'enroulement et/ou comme élément ajustable d'un pré-condenseur (8).

10. Méthode selon la revendication 9, **caractérisée en ce que** le signal pour l'actionnement d'un actionneur est seulement généré si la valeur de réglage a dépassé ou n'a pas atteint une valeur seuil.

11. Méthode selon la revendication 9, **caractérisée en ce qu'**en plus de la teneur en humidité dans la matière de fibres aussi bien la température de la matière de fibres est déterminable.

12. Méthode selon l'une des revendications précédentes 9 à 11, **caractérisée en ce que**, lorsque la valeur de référence est dépassée ou n'est pas atteinte, la vitesse d'enroulement est modifiable.

13. Méthode selon l'une des revendications précédentes 9 à 12, **caractérisée en ce que**, lorsque la valeur de référence est dépassée ou n'est pas atteinte, la pression d'enroulement est modifiable.

14. Méthode selon l'une des revendications précédentes 9 à 13, **caractérisée en ce que**, lorsque la valeur de référence est dépassée ou n'est pas atteinte, la résistance à l'intérieur du pré-condenseur (8) est modifiable.
